# EUROPEAN PATENT APPLICATION

(11) **EP 2 537 507 A2**
(43) Date of publication of application: **26.12.2012**
(21) Application number: 12172720.0
(22) Date of filing: 20.06.2012
(51) Int. Cl.: A61K 6/02, A61K 6/00

(54) **Compositions dentaires contenant des fibres raccourcies**

(30) Priority: 20.06.2011 US 201161499023 P
(71) Applicant: Kerr Corporation, Orange, CA 92857 (US)
(72) Inventor: Kobashigawa, Alvin I, Mission Viejo, California 91765 (US); Tobia, David A, Ladera Ranch, California 92610 (US); Phuong Bui, Vy, Santa Ana, California 92610 (US); Garza, Hector G, Anaheim, California 92610 (US)
(74) Representative: Findlay, Alice Rosemary

(57) **Abstract**

The dental composite includes a resin base and a combination of a short-cut fiber filler and a particulate filler. The short-cut fibers may be made of a glass or a polymer, and may have an average length of about 2 mm to about 6 mm. The particulate filler includes a ground structural filler, which consists of particles having an average particle size of between about 0.05 µm and about 0.5 µm, and a microfiller, which consists of particles having a mean particle size of about 0.04 µm or less. The cured dental composite provides high strength, an improved wear resistance, a dramatically improved fracture resistance and a high gloss retention in simulated extended clinical wear.

## Description

The present invention is generally related to a composite resin material useful for dental restoration, and more particularly to a universal composite resin material which provides high strength, improved wear resistance and high gloss retention in clinical use.

When direct filling composite resins were initially introduced to dentistry, they did not perform well clinically. Within a few years, failures due to excessive wear and bulk fractures occurred, particularly in posterior restorations. Particulate reinforcing fillers used in these restorations improve some physical properties, but these fillers do not sufficiently improve the long-term clinical performance. New developments in particulate filler technology have extended the clinical performance of resins, but failures due to wear and bulk fractures are still of concern.

In an effort to improve clinical performance of composite resins, reinforcing fibers have been introduced into composite resins. Continuous fibers, such as fiberglass and graphite fibers, are routinely used in industry to replace high strength metals. However, continuous fibers are more difficult to work with and require elaborate molding procedures to lay-up and orientate the fibers. In direct filling procedures, the dentist must manipulate and place the restorations quickly into small cavity preparations and cannot use the elaborate molding procedures required for continuous fiber reinforcement. Thus, the use of continuous fibers in dentistry has been limited mostly to indirect restorations, including dentures, which are fabricated outside of the mouth.

To meet the needs of the dentist, products containing short-cut inorganic (e.g., glass) fibers have been introduced, including products such as Restolux™ (Lee Pharmaceuticals, Inc.) and more recently, Alert™ (Jeneric Pentron Inc.). Alert™ was introduced as a "condensable" posterior composite with improved mechanical properties for posterior use. However, during clinical use, negative features in these products have been exposed. Over time, the inorganic fibers become exposed on the surface, which results in a rough, dull surface that wears opposing dentition and irritates oral tissue. Thus, clinical success with short-cut inorganic fibers has also been limited.

The present invention provides a resin-containing dental composition, including a short-cut fiber filler and particulate fillers. The short-cut fiber filler comprises a glass, such as an E-glass or an S-glass, or a polymer, such as a polyester or a polyethylene. The particulate fillers include a ground structural filler consisting essentially of particles having an average particle size of between about 0.05 µm and about 0.5 µm, and a microfiller consisting essentially of particles having a mean particle size of about 0.04 µm or less. Through the use of a combination of short-cut fiber filler and particulate fillers, satisfactory flexural strength and flexural modulus are realized, as well as unexpectedly providing markedly increased fracture resistance in combination with having sustained luster and translucency as required for cosmetic restorations.

In one embodiment, a dental composite is provided that comprises a resin base; a short-cut fiber filler; and particulate fillers ranging from about 10% by volume to about 80% by volume based on the total volume of the dental composite, the particulate fillers consisting essentially of a ground structural filler ranging from about 10% by volume to about 70% by volume based on the total volume of the composite and consisting of ground particles having a mean particle size between about 0.05 µm and about 0.50 µm, wherein the ground structural filler comprises less than 50% by volume of particles above 0.5 µm in diameter; and a microfiller present in an amount up to about 10.0% by volume based on the total volume of the dental composite and consisting of particles having a mean particle size of about 0.04 µm or less, wherein the dental composite after curing has greater than about 25% increase in fracture toughness over an identical sample without the short-cut fiber filler.

In another embodiment, a dental composite is provided comprising a resin base; a short-cut fiber filler present in the resin base in an amount ranging from about 1 part per hundred parts of resin (PHR) to about 25 PHR; and a particulate filler ranging from about 11% to about 80% by volume based on the volume of the dental composite, the particulate filler consisting essentially of a ground structural filler ranging from about 10% to about 70% by volume based on the volume of the dental composite and consisting of ground particles having a mean particle size of between about 0.05 µm and about 0.50 µm, and a microfiller ranging from about 1.0% to about 10.0% by volume based on the volume of the dental composite and consisting of particles having a mean particle size of about 0.04 µm or less, wherein the dental composite after curing has greater than about 25% increase in fracture toughness over an identical sample without said short-cut fiber filler.

In yet another embodiment, a dental composite is provided that comprises a resin base including bis-phenol-A-bis-(2-hydroxy-3-methacryloxypropyl) ether, triethyleneglycol dimethacrylate, and ethoxylated bis-phenol-A-dimethacrylate; a short-cut fiber filler having an average fiber length ranging from about 2 mm to about 6 mm; and a particulate filler ranging from about 11 % to about 80% by volume based on the total volume of the dental composite, the particulate filler consisting essentially of a ground structural filler ranging from about 10% to about 70% by volume based on the total volume of the dental composite and consisting of ground particles having a mean particle size between about 0.05 µm and about 0.50 µm, and a microfiller comprising from about 0.5% to about 5.0% by volume based on the total volume of the dental composite of particles having a mean particle size of about 0.04 µm, and from about 0.5% to about 5.0% by volume based on the total volume of the dental composite of particles having a mean particle size of approximately 0.02 µm, wherein the dental composite after curing has greater than about 25% increase in fracture toughness over an identical sample without said short-cut fiber filler, and wherein the dental composite after curing has an Equilibrium Gloss (G_{E}) at a 60° measurement angle of about 30% or greater after simulated extended clinical wear.

The present invention provides at least in the preferred embodiments a universal composite resin material that provides high strength, improved fracture resistance, improved wear resistance and high gloss retention in clinical use.

The invention will now be further described by way of example with reference to the accompanying drawing:

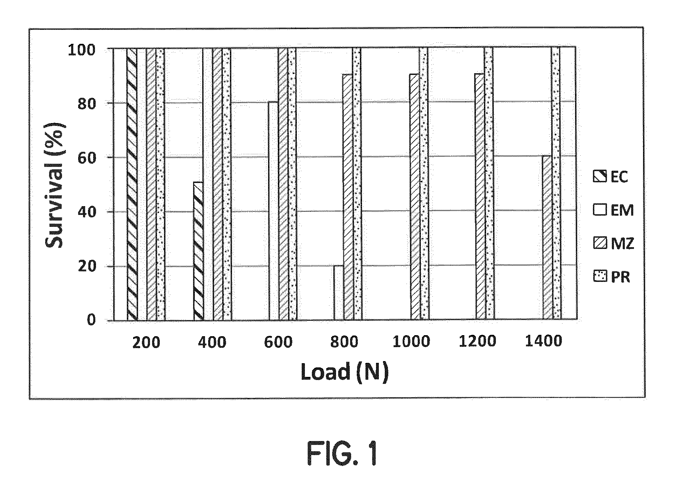

FIG. 1A is a graphical presentation showing survival rates of dental restorations at various loads.

The present invention is directed to a dental composite which includes a resin, a short-cut fiber filler and particulate fillers. According to embodiments of the invention, the resulting dental composites are suitable for use in direct and indirect dental restorations, including but not limited to fillings, orthodontic retainers, bridges, space maintainers, tooth replacement appliances, dentures, crowns, posts, jackets, inlays, onlays, facings, veneers, facets, implants, abutments, cements, bonding agents and splints, which possess optimal physical properties, such as good wear resistance and high strength, and yet maintain high clinical gloss.

According to embodiments of the present invention, a resin-containing dental composition is provided that includes a short-cut fiber filler and particulate fillers. The resin base of the dental composite may include aliphatic or aromatic polymerizable monomers or oligomers. These polymerizable monomers or oligomers comprise a polymerizable component, i.e., at least one polymerizable monomer or prepolymer selected from those known in the art of dental materials, including but not being limited to, resins having (1) free radically-active functional groups, (2) ionically-active functional groups, or (3) both free radically- and ionically-active functional groups. Examples of free radically-polymerizable resins include, but are not limited to those resins with ethylenically unsaturated functional groups such as (meth)acrylates; vinyl monomers such as styrene; vinyl esters such as ethyl vinyl acetate; and a variety of unsaturated cyclic monomers, such as spiro-ortho carbonates, spiro-ortho esters, vinyl cyclic ethers and cyclic acetals.

Polymerizable monomers that are ethylenically unsaturated include those based on acrylic and methacrylic monomers, for example those disclosed in U.S. Patent Nos. 3,066,112, 3,179,623, and 3,194,784 to Bowen; U.S. Patent Nos. 3,751,399 and 3,926,906 to Lee et al.; and U.S. Patent No. 5,276,068 to Waknine. Methacrylate-based monomers are particularly useful, including the condensation product of bisphenol A and glycidyl methacrylate; 2,2'-bis[4-(3-methacryloxy-2-hydroxy propoxy)-phenyl]-propane (BIS-GMA); dipentaerythritol pentaacrylate (DPEPA); pentaerythritol dimethacrylate (PEDM); the condensation product of ethoxylated bisphenol A and glycidyl methacrylate (EBPA-DMA); urethane dimethacrylate (UDMA); ethoxylated bisphenol A di(meth)acrylates including ethoxylated bisphenol A dimethacrylate (EBPDMA) as disclosed in U.S. Patent No. 6,013,694 to Jia, et al.; the condensation product of 2 parts hydroxymethylmethacrylate and 1 part triethylene glycol bis(chloroformate) (PCDMA); polyurethane-based dimethacrylates (PUDMA); and polycarbonate modified-BisGMA (PCBisGMA) and other monomers set forth in U.S. Pat. No. 6,787,629.

The resin base may further comprise additional polymerizable diluent monomers. Such monomers are generally used to adjust the viscosity of the polymerizable composition. Suitable methacrylate-based diluent monomers include, without limitation, hydroxyalkyl methacrylates, such as 2-hydroxyethyl methacrylate, 1,6-hexanediol dimethacrylate, and 2-hydroxypropyl methacrylate; glyceryl dimethacrylate; and ethylene glycol methacrylates, including ethylene glycol methacrylate, diethyleneglycol methacrylate, triethyleneglycol methacrylate and tetraethyleneglycol methacrylate. Triethyleneglycol dimethacrylate (TEGDMA) is exemplary.

Examples of ionically-polymerizable resins include, but are not limited to, vinyl ethers and cyclic monomers such as epoxies, siloranes, lactides, ε-caprolactones and ε-caprolactams.

Examples of resins containing both free radically- and ionically-active functional groups include, but are not limited to the resin oligomers having both an epoxy functionality and a (meth)acrylate functionality, as set forth in commonly owned U.S. Patent No. 7,241,856, which is hereby incorporated by reference.

Additionally, the polymerizable monomers or oligomers may comprise an acid or acid-precursor functional group, such as a carboxylic acid, carboxylic acid anhydride, acyl halide, sulfonic acid, sulfonyl halide, sulfonic anhydride, sulfinic acid, sulfinyl halide, sulfinic anhydride, phosphoric acid, phosphoric acid derivative, phosphonic acid, and phosphonic acid derivative, and combinations thereof. The salts of the corresponding acid may also be used. Exemplary salts include the alkali metal salts.

Exemplary acid-functionalized polymerizable resins include, but are not limited to, acrylic acid, methacrylic acid, 2-(methacryloyloxy)ethyl phosphate, bis(2-(methacryloyloxy)-ethyl) phosphate, biphenyl dimethacrylate, ethylene glycol methacrylate phosphate, 4-methacryloxyethyl trimellitic anhydride, 4-methacryloxyethyl trimellitic acid, adduct reaction product of pyromellitic di-anhydride with 2-hydroxyethylmethacrylate, adduct reaction product of pyromellitic di-anhydride with glycerol dimethacrylate, or adduct reaction product of benzenetetracarboxylic acid di-anhydride with 2-(6-hydroxy-1-oxo-hexyloxy)ethyl methacrylate.

Another suitable class of acid-functionalized polymerizable resin is an ethylenically unsaturated phosphoric acid ester having the general formula:

(CH₂=C(CH₃)CO₂-R-O)ₙP(O)(OH)₃₋ₙ

wherein R is a substituted or un-substituted alkyl or aryl group having about 1 to about 36 carbon atoms and n equals 1 or 2.

According to embodiments of the present invention, the resin base of the dental composite includes bisphenol A glycidyl methacrylate (BISGMA); triethyleneglycol dimethacrylate (TEGDMA); and ethoxylated bis-phenol-A-dimethacrylate (EBADM-6). In another embodiment, the resin base of the dental composite includes bisphenol A glycidyl methacrylate (BISGMA); urethane dimethacrylate (UDMA); ethoxylated bis-phenol-A-dimethacrylate (EBADM-6); and trimethylolpropane trimethacrylate (TMPTA-6).

According to embodiments of the present invention, the resin base is present in the dental composite in an amount of at least 1% by weight and less than 99% by weight, based on the total weight of the dental composite. More particularly, the resin base will typically be present in an amount of at least 10% by weight, for example at least about 15% by weight based on the total weight of the dental composite. The dental composite also typically includes less than about 50% by weight and more typically less than about 40% by weight of the resin base. For example, the resin base will typically be present in a range of about 10% by weight to about 50% by weight, for example from about 15% by weight to about 40% by weight of the overall dental composite.

According to one embodiment of the present invention, the short-cut fiber filler comprises a glass, such as a high-strength glass fiber. As used herein, "high-strength glass" is glass having a tensile strength at 23°C of about 2,400 MPa or greater. For example, the tensile strength may be about 3,000 MPa or greater; about 3,500 MPa or greater; about 4,000 MPa or greater; or about 4,500 MPa or greater at 23°C. Exemplary high-strength glass fibers include A glass, C glass, D glass, E glass, ECRGlass®, AR glass, R glass and S-2 GLASS®. According to one embodiment, the glass short-cut fiber may include an E-glass or an S-glass. According to another embodiment, the glass short-cut fiber may include an S-glass, such as S-2 GLASS®.

The glass short-cut fibers may be obtained from continuous glass fibers cut into discontinuous filaments having a length ranging from about 1 mm to about 6 mm; from about 2 mm to about 6 mm; or from about 3 mm to about 5 mm. For example, the average length of the glass short-cut fiber may be about 1mm, about 2 mm; about 3 mm; about 4 mm; about 5 mm; or about 6 mm. The glass short-cut fibers are not particularly limited to any specific average thickness or average diameter. For example, the glass short-cut fibers may have an average thickness/diameter ranging from about 1 µm to about 500 µm, from about 2 µm to about 250 µm, from about 3 µm to about 200 µm, or from about 4 µm to about 100 µm. In one example, a sample of S-2 GLASS® short-cut fibers may have an average diameter of about 5 µm to about 9 µm.

The glass fibers may be treated with standard coupling agents, such as γ-methacryloxypropyl trimethoxy silane and/or methacrylic anhydride. In one example, S-2 GLASS® short-cut fibers were treated with γ-methacryloxypropyl trimethoxy silane.

According to another embodiment of the present invention, the short-cut fiber filler comprises a fully polymerized thermoplastic material, although a wide variety of polymeric materials may be employed, including partially polymerized thermosetting materials. Thermoplastics allow ease of formability and provide the stiffness, strength, spring back and creep resistance preferable for passive applications. For example, the polymeric material may include polyamides such as nylon; polyesters; glycol esters such as polyethylene terephthalate glycol; polyolefins such as polypropylene or polyethylene; polyimides; polyarylates; polyurethanes; styrene; styrene acrylonitriles; acrylonitrile butadiene styrene (ABS); polysulfones; polyacetals; polycarbonates; polyphenylene sulfides; or a wide variety of other polymeric compositions including vinylesters and epoxy type materials. With respect to obtaining desirable aesthetic appearances, the polymeric fibers may be clear or water white in appearance.

In one embodiment, the polymeric short-cut fiber filler comprises a polytheylene, a polyester or a combination thereof. In another embodiment, the polymeric short-cut fiber filler comprises a high strength, high modulus polyethylene; a high strength, high modulus polyester; or a combination thereof. As used herein, high strength is defined as having a tensile strength from about 10,000 to about 100,000 psi, and high modulus is defined as having a tensile modulus of about 5 GPa or greater, for example from about 5 to about 100 GPa, wherein both of which can be determined by current ASTM methods. According to one embodiment, the polymeric short-cut fiber filler is poly(ethylene terephthalate) (PET) fiber (Engineered Fibers Technology, LLC., Shelton, CT). In yet another embodiment, the polymeric short-cut fiber filler comprises ultra-high molecular weight polyethylene (UHMWPE), which is herein defined as having an average molecular weight (MW_{avg}) greater than about 500,000. An exemplary UHMWPE has a MW_{avg} between about 1 million and about 6 million. For example, according to one embodiment of the invention, the polymeric short-cut fiber filler is SPECTRA® 1000 polyethylene fibers (Honeywell, Inc., Morris Township, NJ).

The polymeric fiber may be modified to enhance bonding to the resin matrix. For example, the surface of the polymeric fiber may be modified by exposing the fiber to plasma treatment, incorporating the fiber through a peroxide initiator, treating the fiber with coupling agents, performing a strong acid oxidation of the fiber, and combinations thereof. In one embodiment, the polymeric fiber is cold gas plasma treated (by Fourth State, Inc., Belmont, CA) to introduce functional groups, such as hydroxyl, carbonyl and carboxyl, to the surface of the fiber and thereby functionalize the surface of the polymeric fiber.

The functionalized surface of the polymeric fiber may be further treated with a coupling agent, which is capable of reacting with at least one of the introduced functional groups and possesses a polymerizable group, to thereby incorporate polymerizable groups onto the surface of the polymeric fiber. In one embodiment, the coupling agent has at least one reactive group and at least one alkenyl functional group. Exemplary coupling agents include, for example, methacrylic anhydride or γ-methacryloxy-propyl trimethoxysilane, both of which are capable of reacting with the hydroxyl group functionality.

While not the subject of this invention and not intending to be bound by any particular theory, the utilization of plasma treatment to enhance the strength of adhesive bonds is a well-proven and documented industrial tool. Typically, plasma treatment is achieved through a vacuum process. The components to be treated are placed in a chamber and the air removed via a vacuum pump to pressures typically less than 100 mTorr. One or more process gases are then flowed into the chamber and allowed to reach equilibrium, typically 100 ― 500 mTorr. Radio-frequency energy supplied to electrodes within the chamber excite the one or more process gases into a plasma. Plasma is a gas comprised of modest concentrations of electrons, ions, as well as other excited meta-stable species. These excited species have sufficient energy to rupture chemical bonds of the polymeric fiber. These ruptured chemical bonds are thermodynamically unstable and combine with gas fragments to normalize their energy, and thereby molecularly re-engineering the surface of the polymeric fiber. Cold gas plasma processes are low energy processes and the species created have little penetrating energy, thus the modification is generally limited to surfaces, e.g., typically no deeper than a few molecular layers.

In one example, a plasma treatment of polyester fibers may be performed with an oxygen/carbon tetrafluoride mixture, which is followed by treatment with methacrylic anhydride. In another example, after a plasma treatment of polyethylene fibers is performed with an oxygen/carbon tetrafluoride mixture, the surface-modified polyethylene fibers are treated with γ-methacryloxy-propyl trimethoxysilane.

The polymeric short-cut fibers may be obtained from continuous fibers cut into discontinuous filaments having a length ranging from about 1 mm to about 6 mm; from about 2 mm to about 6 mm; or from about 3 mm to about 5 mm. For example, the average length of the short-cut fiber may be about 1mm, about 2 mm; about 3 mm; about 4 mm; about 5 mm; or about 6 mm. The polymeric short-cut fibers are not particularly limited to any specific average thickness or average diameter. For example, the polymeric short-cut fibers may have an average thickness/diameter ranging from about 1 µm to about 500 µm, from about 2 µm to about 250 µm, from about 3 µm to about 200 µm, or from about 4 µm to about 100 µm. In one example, a sample of polyester short-cut fibers may have an average diameter of about 5 µm to about 9µm, such as about 8 µm. In another example, a sample of polyethylene short-cut fibers may have an average diameter of about 20 µm to about 40 µm, such as about 30 µm.

The amount of short-cut fiber filler in the dental composite may vary. For convenience, the short-cut fiber filler quantity may be based on parts per hundred parts of resin (PHR). According to some embodiments, the short-cut fiber filler may range from about 1 PHR to about 25 PHR. For example, the short-cut fiber filler may range from about 5 PHR to about 20 PHR, or from about 10 PHR to about 15 PHR.

According to embodiments of the present invention, the dental composite includes particulate fillers, including a ground structural filler and a microfiller. The ground structural filler has a mean particle size at or below the wavelength of light. For example, the mean particle size may be between about 0.05 µm and about 0.50 µm, or between about 0.25 µm and about 0.5 µm. The microfiller has a mean particle size less than about 0.05 µm, for example about 0.02 µm to about 0.04 µm. The particulate fillers used in the composition of the present invention are more fully described in commonly-owned U.S. Pat. No. 6,121,344.

To provide a ground structural filler having a mean particle size of less than 0.5 µm, an extensive comminution step is required. Comminution may be performed in an agitator mill designed to minimize contamination, such as that described in U.S. Pat. No. 6,010,085 entitled "Agitator Mill and Method of Use for Low Contamination Grinding," C. Angeletakis, which is commonly owned and incorporated herein by reference in its entirety. Comminution deagglomerates the structural filler particles by separating particles from clusters, decreases the size of the structural filler particles, eliminates large particles by breakage and increases the specific surface area of the structural filler particles by producing a large quantity of very fine, non-spherical particles. Size reduction with an agitator mill occurs due to a combination of impact with the milling media, abrasion with the milling media and attrition of the particles.

Structural fillers suitable for use in the present invention include barium magnesium aluminosilicate glass, barium aluminoborosilicate glass, amorphous silica, silica-zirconia, silica-titania, barium oxide, quartz, alumina and other inorganic oxide particles.

A microfiller is further included as a particulate filler in the dental composite. Microfillers include fumed silica and other inorganic particles characterized as having a mean particle size of about 0.05 µm or less. For example, microfillers according to embodiments of the present invention may have a mean particle size of about 0.04 µm or less. Examples of microfillers include products available under the trade designations AEROSIL® OX-50, AEROSIL® 130, AEROSIL® 150, and AEROSIL® 200 available from DeGussa AG, (Hanau, Germany) and CAB-O-SIL® TS-530 available from Cabot Corp. (Tuscola, Ill.).

According to one embodiment of the present invention, the dental composite comprises a particulate filler ranging from about 10% by volume to about 80% by volume based on the total volume of the dental composite, the particulate filler consisting essentially of: a ground structural filler ranging from about 10% by volume to about 70% by volume based on the total volume of the composite and consisting of ground particles having a mean particle size between about 0.05 µm and about 0.50 µm, wherein the ground structural filler comprises less than 50% by volume of particles above 0.5 µm in diameter; and a microfiller present in an amount up to about 10.0% by volume based on the total volume of the dental composite and consisting of particles having a mean particle size of about 0.04 µm or less.

According to another embodiment of the present invention, the dental composite comprises a particulate filler ranging from about 11 % to about 80% by volume based on the volume of the dental composite, the particulate filler consisting essentially of: a ground structural filler ranging from about 10% to about 70% by volume based on the volume of the dental composite and consisting of ground particles having a mean particle size of between about 0.05 µm and about 0.50 µm, and a microfiller ranging from about 1.0% to about 10.0% by volume based on the volume of the dental composite and consisting of particles having a mean particle size of about 0.04 µm or less.

According to yet another embodiment, the dental composite comprises a particulate filler ranging from about 11 % to about 80% by volume based on the total volume of the dental composite, the particulate filler consisting essentially of: a ground structural filler ranging from about 10% to about 70% by volume based on the total volume of the dental composite and consisting of ground particles having a mean particle size between about 0.05 µm and about 0.50 µm, and a microfiller comprising from about 0.5% to about 5.0% by volume based on the total volume of the dental composite of particles having a mean particle size of about 0.04 µm, and from about 0.5% to about 5.0% by volume based on the total volume of the dental composite of particles having a mean particle size of approximately 0.02 µm.

The dental composite may further include a polymerization initiator system. In certain embodiments, the dental composites are chemically polymerizable, i.e., the compositions contain a chemically polymerizable component and a chemical initiator (i.e., initiator system) that can polymerize, cure, or otherwise harden the composition without dependence on irradiation with actinic radiation. Such chemically polymerizable compositions are sometimes referred to as "self-cure" compositions and may include glass ionomer cements, resin-modified glass ionomer cements, redox cure systems, and combinations thereof. A common redox initiator system used in self-cure or dual-cure resin contains a benzoyl peroxide (BPO) catalyst and a tertiary amine activator. Another self-cure initiator, especially suitable for heat and pressure curable compositions, include heat cure initiators such as benzoyl peroxide and 1,1'-azobis(cyclohexanecarbonitrile).

In certain embodiments, the dental composites are photopolymerizable, i.e., the compositions contain a photopolymerizable component and a photoinitiator (i.e., a photoinitiator system) that upon irradiation with actinic radiation initiates the polymerization (or hardening) of the composition. Such photopolymerizable compositions can comprise free radically polymerizable photoinitiator systems suitable for light-curing the polymeric material. The light cure system may be selected from known light-activated polymerization initiators, including but not being limited to benzil, benzoin, benzoin methyl ether, DL-camphorquinone (CQ) and benzil diketones. Either UV-activated cure or visible light-activated cure (about 230 nm to about 750 nm) is acceptable. The amount of photoinitiator is selected according to the curing rate desired. Additionally, a plurality of photoinitiators may be used. A minimal catalytically-effective amount of photoinitiator is generally about 0.01 % by weight based on the total weight of the polymerizable components. Faster rates of cure are achieved with amounts of catalyst in the range from greater than about 0.01 % to about 5% by weight based on the total weight of the polymerizable components. Visible light curing systems may further comprise polymerization accelerators, which include various organic tertiary amines that are well known in the art. In visible light curable compositions, the tertiary amine may be an acrylate derivative, such as dimethylaminoethyl methacrylate or diethylaminoethyl methacrylate (DEAME), or an aromatic tertiary amine, such as ethyl dimethylamino benzoate (EDMAB), present in amounts ranging from about 0.05 to about 2 weight percent based on the total weight of the polymerizable components, for example, from about 0.1 to about 0.5 weight percent.

In certain embodiments, the dental composites are both self-curable and photocurable, *i.e*., dual curable. For example, according to some embodiments, the dental composites include both a polymerization initiator of the photoinitiator type and a self-cure initiator type. One exemplary dual curable dental composite comprises both camphorquinone (CQ) and 1,1'-azobis(cyclohexanecarbonitrile).

The dental composite may also comprise other additives and solvents known in the art, for example, ultra-violet light absorbers, anti-oxidants such as BHT, stabilizers, fillers, pigments, opacifiers, handling agents/rheology modifiers, fluorescence agents, thermochromic dyes and others. It is useful to employ an ultraviolet absorber in amounts ranging from about 0.05 to about 5.0 weight percent. Such UV absorbers are particularly desirable in these visible light curable compositions in order to avoid discoloration of the resin from any incident ultraviolet light. Suitable UV absorbers are the various benzophenones, particularly UV-9 and UV-5411 available from American Cyanamid Company, and benzotriazoles known in the art, particularly 2-(2'-hydroxy-5'-methylphenyl)-benzotriazole, sold under the trademark TINUVIN® P by Ciba-Geigy Corporation, Ardsley, N.Y.

According to embodiments of the present invention, the cured dental composite, obtained after curing a composition comprising the resin base, the short-cut fiber filler and the particulate filler, as described above, has greater than about 25% increase in fracture toughness over an identical sample without said short-cut fiber filler. For example, the fracture toughness may be increased by an amount that is greater than about 50%, greater than about 100%, or greater than about 200% over an identical sample without said short-cut fiber filler.

According to embodiments of the invention, the cured dental composite, obtained after curing a composition comprising the resin base, the short-cut fiber filler and the particulate filler, as described above, has an Equilibrium Gloss (G_{E}) value at a 60° measurement angle of about 30% or greater after simulated extended clinical wear. The G_{E} value represents a final gloss state after being subjected to simulated extended clinical wear testing, as described below. In one embodiment, the cured dental composite has a G_{E} value at a 60° measurement angle of about 40% or greater after simulated extended clinical wear.

This invention is illustrated by the following examples that are merely for the purpose of illustration and are not to be regarded as limiting. For convenience, individual component amounts are provided as weight percents that are based on the total weight of the composition. However, weight percentage may be converted to an approximate volume percentage based on the densities provided in Table 1 below. Alternatively, the individual components may be apportioned as a volume percent that is based on the total volume of the composition.

**Table 1: Densities of individual components.**

| Material | Density¹ |
|---|---|
| Polyester (Engineered Fibers Inc.) | 1.4² |
| Polyethylene (Spectra® 1000) | 0.97² |
| S-2 GLASS® (Engineered Fibers Inc.) | 2.48² |
| Belleglass™ Translucent Dentin Filler | 2.46 (0.01) |
| Belleglass™ Opaceous Dentin Filler | 2.63 (0.01) |
| Premise™ Treated Filler | 2.52 (0.01) |
| 0.4 Micron Treated Filler | 2.91 (0.01) |
| OX-50 Fumed Silica | 2.4 (0.01) |
| TS-530 Fumed Silica | 2.28 (0.01) |
| Belleglass™ Resin | 1.21 (0.01) |
| CAD/CAM Resin | 1.16 (0.01) |

| | |
|---|---|
| 1. Densities were determined using Micromiretics AccuPyc 1330, unless otherwise noted, and are reported in grams per cubic centimeter with the standard deviation in parentheses. 2. Density provided by the manufacturer. | |

### EXAMPLES

For the following examples, two commercial resins were utilized. Belleglass™ Resin and CAD/CAM Resin formulations are shown in Table 2.

**TABLE 2: Resin Formulations**

| Belleglass™ Resin | | |
|---|---|---|
| Abbreviation | Component | Weight Percent (%) |
| BIS-GMA | Bisphenol A Glycidyl Methacrylate | 36.8 |
| TEGDMA | Triethylene Glycol Dimethacrylate | 24.0 |
| EBADM | Ethoxylated Bisphenol A Dimethacrylate | 36.8 |
| UV-9 | Hydroxymethoxybenzophenone | 0.58 |
| BHT | Butylhydroxy Toluene | 0.05 |
| CQ | Camphorquinone | 0.17 |
| Vazo 88 | Azobis(cyclohexanecarbonitrile) | 1.1 |
| DMAEMA | Dimethylaminoethyl Methacrylate | 0.5 |
| | | |

| CAD/CAM Resin | | |
|---|---|---|
| Abbreviation | Component | Weight Percent (%) |
| BIS-GMA | Bisphenol A Glycidyl Methacrylate | 25.0 |
| UDMA | Urethane Dimethacrylate | 10.0 |
| EBADM-6 | Ethoxylated Bisphenol A Dimethacrylate (SR541) | 58.95 |
| TMPTA-6 | Trimethylol Bisphenol A Dimethacrylate (SR499) | 5.0 |
| BHT | Butylhydroxy Toluene | 0.05 |
| Vazo 88 | Azobis(cyclohexanecarbonitrile) | 1.0 |

### EXAMPLE 1

An exemplary formulation using Belleglass™ Translucent Dentin Resin with 10 PHR polymeric short-cut fiber filler includes about 24.3 wt% Resin, 2.4 wt% fiber filler and 73.3 wt% particulate filler, wherein the wt% is based on the total weight of the composite. To determine the effect, if any, of varying the length and amount of polymeric short-cut fiber filler, various samples were prepared. Various lengths of polyester short-cut fiber (Engineered Fibers Inc.) were cold gas plasma treated (Fourth State Inc.) to produce hydroxyl and carboxyl functionality, treated with methacrylic anhydride (5%) in tetrahydrofuran, filtered and vacuum dried at 80°C for 6 hrs. The fibers were mixed into Belleglass™ Translucent Dentin Resin in concentrations from 0 to 20 PHR (Parts Per Hundred Resin) and compounded into pastes with Belleglass™ Translucent Dentin Filler, which includes the particulate filler described in commonly-owned U.S. Patent No. 6,121,344 by Angeletakis et al. Specifically, the ground structural filler has an average particle size of 0.4 µm, referred to as the 0.4 µm filler. The formulations were then cured per the manufacturer's instructions and tested for fracture toughness (K_{Ic}), flexural strength and flexural modulus according to the Single Edge Notched Beam Method (SENB) and ISO 4049, respectively. The results are shown in Table 3, with standard deviation (s.d.) in parentheses.

**TABLE 3: Belleglass™ Translucent Dentin Resin and Filler with Polyester Short-cut Fibers**

| Fiber Length (mm) | Fiber amount (PHR) | K_{Ic}, MPa•m^{1/2} (s.d.) | Flex. Strength MPa (s.d.) | Flex. Modulus GPa (s.d.) |
|---|---|---|---|---|
| n/a | 0 | 1.18 (0.1) | 149 (18) | 10.5 (1.3) |
| 2 | 10 | 1.27 (0.1) | 110 (9) | 9.5 (0.6) |
| 2 | 15 | 1.93 (0.3) | 110 (9) | 9.5 (0.4) |
| 2 | 20 | 2.23 (0.6) | 119 (11) | 9.4 (0.2) |
| 5 | 10 | 1.42(0.1) | 113 (11) | 9.9 (0.3) |
| 5 | 15 | 2.11 (0.3) | 120 (12) | 10.0 (0.5) |
| 5 | 20 | 2.76 (0.2) | 114 (22) | 9.3 (0.3) |

The formulation without polymeric short-cut fiber filler was used as a control sample. The Belleglass™ Translucent Dentin Resin and filler formulations that included polyester short-cut fiber filler demonstrated an increase in the fracture toughness as the length of the fiber and amount of the fiber filler increased. The increase in fracture toughness was quite dramatic, approaching up to three times the value of the control sample with 5 mm fiber at 20 PHR fiber filler content. The flexural strength and modulus both observed a decrease relative to the control, with 5 mm fiber at 15 PHR showing the lowest reduction in value. The length of the fiber affects the fiber filler loading and the handling qualities of the pastes. In general, the longer the fiber, the lower the fiber filler loading and the poorer the handling quality of the paste. In these examples, the 2 mm length at 15 to 20 PHR provided a favorable combination of fracture toughness, filler loading and paste handling qualities.

### EXAMPLE 2

Another formulation of the indirect composite resin, Belleglass™ Translucent Dentin, was prepared. Various amounts and lengths of polyethylene short-cut fiber (Spectra® 1000, Honeywell Corp.) were cold gas plasma treated under the same conditions used in Example 1. The surface modified fibers were subsequently treated with γ-methacryloxypropyl trimethoxy silane, A-174 (OSI Specialties, Inc.) as a 10% methanolic solution, filtered, and vacuum dried at 80 °C for 6 hrs. The dried fibers were then mixed into Belleglass™ Translucent Dentin Resin, in lengths of 1 mm to 5 mm and in amounts of 0 to 25 PHR, and compounded into pastes with Belleglass™ Translucent Dentin Filler. The formulations were cured per the manufacturer's instructions and tested for fracture toughness, flexural strength and modulus according to the Single Edge Notched Beam Method (SENB) and ISO 4049, respectively. The results are shown in Table 4, with standard deviation (s.d.) in parentheses.

**TABLE 4: Belleglass™ Translucent Dentin Resin and Filler with Polyethylene Short-cut Fibers**

| Fiber Length (mm) | Fiber amount (PHR) | K_{Ic} MPa•m^{1/2} (s.d.) | Flex. Strength MPa (s.d.) | Flex. Modulus GPa (s.d.) |
|---|---|---|---|---|
| n/a | 0 | 1.1 (0.1) | 150 (30) | 11.1 (0.2) |
| 1 | 15 | 1.63 (0.48) | 83 (5) | 8.1 (0.3) |
| | 15 | 1.39 (0.10) | 80.6 (15.4) | 8.7 (0.8) |
| | 15 | 1.30 (0.15) | 79.2 (6.1) | 8.2 (0.6) |
| | 20 | 1.66 (0.19) | 84 (4) | 7.9 (1.0) |
| | 20 | 1.62 (0.3) | 78.9 (7.3) | 7.9 (0.7) |
| | 20 | 1.52(0.11) | 78.8 (4.0) | 7.9 (0.9) |
| | 25 | 1.96 (0.21) | 80.6 (15.4) | 8.7 (0.8) |
| 3 | 5 | 1.64 (0.31) | 118 (22) | 11.3 (0.5) |
| | 10 | 1.63 (0.09) | 142 (29) | 10.6 (0.6) |
| | 15 | 1.87 (0.32) | 136 (14) | 11.0 (0.7) |
| | 15 | 1.73 (0.19) | 128 (10) | 9.7 (0.6) |
| | 20 | 2.20 (0.20) | 126 (31) | 11.4 (0.8) |
| 5 | 10 | 1.97 (0.54) | 97 (11) | 7.7 (0.5) |
| | 15 | 4.18 (0.73) | 91 (16) | 7.6 (1.0) |

A formulation without polymeric short-cut fiber filler was used as the control. The Belleglass™ Translucent Dentin Resin and filler formulations that include polyethylene short-cut fiber filler demonstrated an increase in the fracture toughness as the length of the fiber and amount of the fiber filler increased. The increase in fracture toughness was also dramatic, reaching almost four times the control with 5 mm fiber at 15 PHR fiber filler content. The flexural strength and modulus was reduced, similar to Example 1, although the flexural strength and flexural modulus were not significantly reduced with 3 mm fibers at 10 to 15 PHR. It was observed that the length of the fiber affects the fiber filler loading and the handling qualities of the pastes. For example, as the fiber length increases, the fiber filler loading decreases and the handling qualities become poorer. Overall, 3 mm length fiber in amounts of 10 to 15 PHR provided a favorable combination of fracture toughness, filler loading and paste handling qualities.

### EXAMPLE 3

The formulation in Example 2 was applied to Computer Aided Design/Computer Aided Manufacturing (CAD/CAM) processing. Several formulations containing 5 mm fibers were evaluated in processed blocks. The formulations were compression molded into blocks at 100 °C while under 5,000 psi of pressure for 1 hour. The cured specimens were machined using a diamond saw for testing. The data is summarized in Table 5, with standard deviation (s.d.) in parentheses.

**TABLE 5: Belleglass™ Translucent Dentin Resin and filler with Polyethylene Short-Cut Fiber, CAD/CAM Processed**

| Fiber Length (mm) | Fiber amount (PHR) | K_{Ic} MPa•m^{1/2} (s.d.) | Flex. Strength MPa (s.d.) | Flex. Modulus GPa (s.d.) |
|---|---|---|---|---|
| n/a | 0 | 1.04 (0.04) | 142 (10) | 13.1 (0.7) |
| 5 | 10 | 1.85 (0.4) | 94 (15) | 7.1 (0.9) |
| | 15 | 2.29 (0.81) | 84 (8.0) | 7.0 (0.7) |

The formulation in Table 5 without short-cut fiber filler was used as a control sample. In general, the fracture toughness was again improved with increasing fiber filler content, but other physical properties did not show an improvement over the Belleglass™ processing samples of Example 2.

### EXAMPLE 4

The formulation in Example 2 was applied to Computer Aided Design/Computer Aided Manufacturing (CAD/CAM) processing. A formulation containing 3 mm S-2 GLASS® fibers was evaluated in processed blocks. The formulation was compression molded into a block at 100 °C while under 5,000 psi of pressure for 1 hour. The cured specimen was machined using a diamond saw for testing. The data is summarized in Table 5, with standard deviation (s.d.) in parentheses. The formulation in Table 6 without short-cut glass fiber filler was used as a control sample.

**TABLE 6: Belleglass™ Translucent Dentin Resin and filler with S-2 GLASSL® Fiber, CAD/CAM Processed**

| Fiber Length (mm) | Fiber amount (PHR) | K_{Ic} MPa•m^{1/2} (s.d.) | Flex. Strength MPa (s.d.) | Flex. Modulus GPa (s.d.) |
|---|---|---|---|---|
| n/a | 0 | 1.04 (0.04) | 142 (10) | 13.1 (0.7) |
| 3.0 | 20 | 2.14 (0.2) | 151 (17) | 12 (1.3) |

In general, the fracture toughness was again improved by the addition of short-cut fiber filler, but other physical properties did not show any appreciable improvement over the Belleglass™ processing sample without short-cut glass fiber filler.

### EXAMPLE 5

Example 5 is a formulation of a high strength indirect resin, Belleglass™ Opaceous Dentin containing the polyethylene short-cut fibers (Spectra® Fiber 1000) formulated in 3 mm lengths. The short-cut fiber filler was compounded into Belleglass™ Opaceous Dentin Resin and mixed into a paste with Belleglass™ Opaceous Dentin Treated Filler, which is a 4.9 µm sized particle filler. The dental compositions were cured per the manufacturer's instructions and tested for fracture toughness, flexural strength and modulus according to the Single Edge Notched Beam Method (SENB) and ISO 4049, respectively. Table 7 summarizes the data, with standard deviation (s.d.) in parentheses, for the Opaceous Dentin formulation with 3 mm Spectra® 1000 fibers.

**TABLE 7: Belleglass™ Opaceous Resin With Polyethylene Short-Cut Fiber**

| Fiber Length (mm) | Fiber amount (PHR) | K_{Ic} MPa•m^{1/2} (s.d.) | Flex. Strength MPa (s.d) | Flex. Modulus GPa (s.d.) |
|---|---|---|---|---|
| n/a | 0 | 1.48 (0.1) | 158 (11) | 21.0 (1.4) |
| 3 | 5 | 2.05 (0.22) | 146 (16) | 18.7 (0.7) |
| | 10 | 2.50 (0.30) | 139 (14) | 17.2 (0.7) |
| | 15 | 3.08 (0.57) | 157 (8.0) | 16.8 (1.2) |
| | 20 | 2.58 (0.21) | 148 (14) | 17.1 (13) |

Similar to that observed in previous examples, the fracture toughness was increased relative to the control, with a maximum observed at about 15 PHR that is more than twice the control sample value. The Belleglass™ Opaceous Dentin formula demonstrates higher physical property values compared to the Translucent Dentin formula, as can be seen by comparing the control samples. Additionally, the Opaceous Dentin composition has a coefficient of thermal expansion that matches human dentin. Generally, Opaceous Dentin compositions are designed to be used as a sublayer in a restoration, such as onlays, crowns or bridges, to replace human dentin. In the foregoing samples, the fracture toughness exceeds that of human dentin and the flexural strength and modulus remain high. However, because of the conventionally large particle hybrid filler of the Opaceous Dentin formulation, it is not readily polishable and therefore, is generally only suitable for use in a sublayer of a dental restoration.

### EXAMPLE 6

Example 6 is a formulation for a direct filling composite resin, Premise™, also containing Spectra® 1000 polyethylene short-cut fibers. The fiber was incorporated into Premise™ Resin and formulated into paste with Premise™ Filler, which includes 0.4 µm ground filler. Because Premise™ resin is photocurable, all samples were light cured for 40 seconds with an Optillux 501 Light calibrated to greater than 500 MW/cm² output. The cured samples were tested for fracture toughness, flexural strength and modulus according to the Single Edge Notched Beam Method (SENB) and ISO 4049, respectively. The results are shown in Table 8 with standard deviation (s.d.) in parentheses.

**TABLE 8: Premise™ Direct Filling Resin with Polyethylene Short-Cut Fiber**

| Fiber Length (mm) | Fiber amount (PHR) | K_{Ic} MPa•m^{1/2} (s.d.) | Flex. Strength MPa (s.d.) | Flex. Modulus GPa (s.d.) |
|---|---|---|---|---|
| n/a | 0 | 1.13 (0.1) | 105 (14) | 8.5 (0.4) |
| 3 | 20 | 1.89 (0.1) | 128 (11) | 8.0 (0.4) |
| 5 | 20 | 1.85 (0.2) | 116 (15) | 9.6 (0.4) |

For the Premise™ direct filling dental compositions comprising the polymeric short-cut fiber filler, the strengths were generally improved over the control, but the fiber filler loading and handling qualities deteriorated as the fiber length increased. Similar to several other examples, the 3 mm polyethylene short-cut fiber filler provided a favorable combination of fracture toughness, filler loading and paste handling qualities.

### EXAMPLE 7

Various CAD/CAM block formulas were evaluated for fatigue resistance. According to one embodiment of the present invention, an exemplary formulation (PR) using CAM/CAD resin with 20 PHR glass short-cut fiber filler is shown in Table 9. The PR formulation was tested in comparison to commercial products MZ100 (MZ), a composite block sold by 3M Corporation, and Empress CAD (EC) and E-Max (EM), two porcelain products sold by Ivoclar Vivadent Inc. Briefly, standardized crown restorations were milled by the Cerec 3 system (Sirona Dental Systems, Inc.), cemented to a tooth and subjected to loads from 400 to 1,400 N for up to 185,000 cycles.

More specifically, each specimen was stored in distilled water at ambient temperature for at least 24 hours following adhesive restoration placement. Masticatory forces were then simulated with an artificial mouth using closed-loop servohydraulics (Mini Bionix II, MTS Systems). Each specimen was placed into the load chamber and situated with a sliding table positioning device. The chewing cycle was simulated by an isometric contraction applied through a stainless steel sphere with a diameter of 7 mm. Because of the standardized occlusal anatomy, all specimens could be adjusted, through the same positioning device, in the same reproducible position with the sphere contacting the mesiobuccal, mesiolingual, and distobuccal cusps thereby providing a tripod contact. The load chamber was filled with distilled water to submerge the sample during testing.

Cyclic load was applied at a frequency of 5 Hz, starting with a load of 200 N for 5,000 cycles, which provides a preconditioning phase of the experiment, followed by stages of 400, 600, 800, 1000, 1200, and 1400 N at a maximum of 30,000 cycles each. Samples were loaded until fracture or to a maximum of 185,000 cycles. The number of endured cycles was recorded and the survival rate, based on 185,000 cycles, was calculated for each sample.

**TABLE 9: CAD/CAM Formulation with S-2 GLASS® Short-Cut Fiber.**

| Component | Weight % |
|---|---|
| CAD/CAM Resin¹ | 25.0 |
| S-2 GLASS® Short-cut Fiber² | 5.0 |
| Microfiller³ | 3.8 |
| Ground Particulate Filler⁴ | 66.2 |

| | |
|---|---|
| 1. Resin formula provided in Table 2 2. 3 mm fibers, treated with γ-methacryloxypropyl trimethoxy silane 3. TS-530 Fumed Silica 4. Treated 0.4 µm filler | |

The comparison data for the CAD/CAM block samples, PR, MZ, EC and EM, is illustrated in FIG. 1. The porcelain EC and EM composites did not survive at 600 and 1000 N loads, respectively. The composite MZ had a 60% survival rate at 1,400 N. Unexpectedly, the PR sample had a 100% survival rate at 1,400 N. Therefore, the combination of S-2 GLASS® short-cut fiber filler and particulate filler provide a dramatic and unexpected increased fatigue resistance over other commercial composites.

### EXAMPLE 8

GLOSS TESTING: The improvement in physical properties of the dental composites by the presence of fiber filler in the composite is known to come at the consequence of introducing negative effects. As described above, over time and through normal wear, fibers may become exposed on the surface, which results in a rough, dull surface that wears opposing dentition and may irritate oral tissue. To evaluate whether the dental compositions according to the present invention would also suffer from the expected deterioration of gloss, several indirect dental restorative compositions were prepared using Premise Indirect™, which is a universal nano-filled dental restorative composite. Premise Indirect™ composite includes the particulate filler described in commonly-owned U.S. Patent No. 6,121,344 by Angeletakis et al. Specifically, the ground structural filler has an average particle size of 0.4 µm, referred to as the 0.4 µm filler. In select samples, short-cut fiber filler was added to the Premise Indirect Translucent Dentin™ composite and subsequently cured according to the manufacturer's instructions. A sample of commercially-available Alert™ composite, which comprises glass fibers that are fused to particulate filler, was prepared as a control sample as well as Premise Indirect Primary Dentin™, which contains larger particles (4.9 µm). The gloss of the cured composites was measured before and after the toothbrush wear testing, as further described below. The equilibrium gloss results are shown in Table 10 below, with standard deviation (s.d.) in parentheses.

**TABLE 10: Comparison of Equilibrium Gloss (G_{E}) of Dental Composite Formulations.**

| DESCRIPTION | | Particle filler size (µm) | G_{E}, (s.d.) |
|---|---|---|---|
| Premise™ Indirect Translucent Dentin | | 0.4 | 43 (8) |
| | (20 PHR 3mm Spectra^{®} 1000 in resin) | | |
| Premise™ Indirect Translucent Dentin | | 0.4 | 46 (8) |
| | (20 PHR 5mm Polyester in resin) | | |
| CAD/CAM Resin | | 0.4 | 36 (8) |
| | (20 PHR, 3mm S-2 GLASS® Fiber in resin) | | |
| Premise™ Indirect Translucent Dentin Control | | 0.4 | 49 (5) |
| | (no fiber) | | |
| Premise™ Indirect Primary Dentin Control | | 4.9 | 14 (2) |
| | (no fiber, large particle size) | | |
| Alert™ Control | | 0.86 | 15 (2) |
| | (glass fiber fused to particle) | | |

To simulate extended clinical wear, the following tooth brush wear test was developed. Each sample was cured and polished according to the manufacturer's instructions and conditioned in water at 37 °C for 14 days prior to testing. Toothbrush wear testing was conducted as follows: A sample material was seated into a jig designed to fit onto a rotating turntable, cured and polished according to manufacturer's instructions. The jig was set into the turntable rotated at 150 rpm. The wall of the turntable was wrapped in tape to form a bath to hold a dentrifice (Crest, Cincinnati, OH) diluted with water. An automatic toothbrush (Braun, Model #4729, Germany) was swept over the sample surface and a 250-260 gram-force load was applied to the head. Each sample was tested until the surface gloss of the sample reached a constant equilibrium state, which became apparent after about 16 hours of toothbrushing and was measured off a curve derived from plotting the toothbrush gloss value versus time.

The inventors have previously discovered that high clinical gloss may be predicted from the toothbrush wear test described above. Surface gloss deteriorates with toothbrush wear according to the equation: G = G_{E} + be^{-x}, where G is the gloss value as measured in percent (%) at 60°, G_{E} is the Equilibrium Gloss value, b is a constant that is material dependent, e is Euler's constant, and x is time (hours). The Equilibrium Gloss represents a final gloss state after being subjected to the aforementioned toothbrush wear test. In general, G_{E} values of about 30% or greater provide acceptable clinical performance. For example, G_{E} values of about 40% or greater retain a high gloss after the simulated toothbrush wear test, whereas G_{E} values at about 20% are dull in appearance after the simulated toothbrush wear test.

A MICRO-TRI-GLOSS® glossmeter (BYK-Gardner, Columbia, MD) was used to collect photoelectric measurements of specularly reflected light from the sample surface before brushing (*i.e.,* initial gloss, G_{I}), and after toothbrush wear testing (*i.e.,* equilibrium gloss, G_{E}). Specularly reflected light from the surface was measured at 60° geometry. The measuring pot was fitted with a black paper port (89 mm x 35 mm) to fit onto the test samples. The glossmeter was calibrated per the manufacturer's instructions, set for 30 seconds measuring time, and inverted to allow placement of the sample and a black paper background. A 100 gram weight was applied to the sample to assure adequate adaptation to the inverted glossmeter. Each sample was then measured four times at 90° rotation for a total of 16 measurements for each material. The mean gloss and standard deviation of each sample is shown in Table 10.

As shown in Table 10, the G_{E} value may be affected by the filler composition. The first two Premise™ Indirect compositions comprise two different polymeric short-cut fibers. The G_{E} value of both of these formulations remained high. A CAD/CAM resin composition was prepared using S-2 GLASS® short-cut fibers, which still maintained high gloss. Another Premise™ Indirect composition was prepared as a control sample with no fibers, which revealed that the polymeric short-cut fibers have minimal effect on the G_{E} value. Another sample of Premis™ Indirect formula included a large particle size filler (4.9 µm), as a negative control, that showed a markedly reduced G_{E} value. The final comparative sample was a commercial product, Alert™, which contains both large particulate filler and glass short-cut fibers (negative control). The Alert™ sample showed a markedly reduced G_{E} value.

Thus, the dental composites comprising a resin, a short-cut fiber filler, and particulate fillers, including a ground structural filler and a microfiller, according to embodiments of the present invention, provide a restoration having the high strength useful for load bearing restorations and also provide translucency and surface gloss, which are useful in cosmetic restorations. Through the use of glass or polymeric short-cut fiber filler, the composite demonstrated an unexpected increase in the fracture resistance, reaching as high as about 4 four times the control sample. The particulate filler particles having a mean particle size less than the wavelength of light permit the retention of the luster and translucency of dispersion reinforced composites. Thus, through the appropriate combination of short-cut fiber filler and particulate filler, a dental composite material has been realized that provides high strength, improved fracture resistance, improved wear resistance and high gloss retention.

While the present invention has been illustrated by the description of one or more embodiments thereof, and while the embodiments have been described in considerable detail, additional advantages and modifications will readily appear to those skilled in the art.

## Claims

1. A dental composite, comprising:
a resin base;
a short-cut fiber filler; and
a particulate filler ranging from 10% by volume to 80% by volume based on the total volume of the dental composite, the particulate filler consisting essentially of:
a ground structural filler ranging from 10% by volume to 70% by volume based on the total volume of the composite and consisting of ground particles having a mean particle size between 0.05 µm and 0.50 µm, wherein the ground structural filler comprises less than 50% by volume of particles above 0.5 µm in diameter; and
a microfiller present in an amount up to 10.0% by volume based on the total volume of the dental composite and consisting of particles having a mean particle size of 0.04 µm or less.

2. The dental composite of claim 1, wherein the short-cut fiber filler is short-cut glass fiber.

3. The dental composite of claim 2, wherein the short-cut glass fiber is a high-strength glass fiber having a tensile strength at 23°C of 2,400 MPa or greater.

4. The dental composite of claim 2 or 3, wherein the short-cut glass fiber is E-glass or S-glass fiber.

5. The dental composite of claim 1, wherein the short-cut fiber filler comprises a polymer selected from the group consisting of polyesters and polyethylenes.

6. The dental composite of claim 5, wherein the polymer is a high strength, high modulus polymer, wherein the polymer has a tensile strength from 10,000 to 100,000 psi and a tensile modulus of 5 GPa or greater.

7. The dental composite of claim 5 or 6, wherein the polymer is a cold gas plasma treated polymer.

8. The dental composite of claim 7, wherein the cold gas plasma treated polymer is surface treated with a coupling agent having at least one alkenyl functional group.

9. The dental composite of claim 8, wherein the coupling agent is methacrylic anhydride or γ-methacryloxy-propyl trimethoxysilane.

10. The dental composite of any preceding claim, wherein the short-cut fiber filler has an average length ranging from 2 mm to 6 mm, preferably from 3 mm to 5 mm.

11. The dental composite of any preceding claim, wherein the short-cut fiber has an average diameter within the range of from 1 µm to 500 µm.

12. The dental composite of any preceding claim, wherein the dental composite after curing has greater than 25% increase in fracture toughness over an identical sample without said short-cut fiber filler, preferably greater than 50% increase in fracture toughness over an identical sample without said short-cut fiber filler, more preferably greater than 100% increase in fracture toughness over an identical sample without said short-cut fiber filler.

13. The dental composite of any preceding claim, wherein the dental composite after curing has an Equilibrium Gloss (G_{E}) value at a 60° measurement angle of 30% or greater after simulated extended clinical wear, preferably 40% or greater after simulated extended clinical wear.

14. The dental composite of any preceding claim, wherein the polymeric short-cut fiber filler is present in the resin base in an amount ranging from 1 part per hundred parts of resin (PHR) to 25 PHR, more preferably from 5 PHR to 20 PHR.

15. The dental composite of any preceding claim, wherein the resin base includes bis-phenol-A-bis-(2-hydroxy-3-methacryloxypropyl) ether; triethyleneglycoldimethacrylate; and ethoxylated bis-phenol-A-dimethacrylate.
